(19) Europäisches Patentamt
European Patent Office
Office européen des brevets

(11) EP 2 617 739 B1

# (12) EUROPEAN PATENT SPECIFICATION

(45) Date of publication and mention of the grant of the patent:
**29.04.2020 Bulletin 2020/18**

(21) Application number: **11824428.4**

(22) Date of filing: **09.09.2011**

(51) Int Cl.:
*C08F 4/02* (2006.01) *C08F 4/654* (2006.01)
*C08F 10/00* (2006.01) *C07C 31/28* (2006.01)
*C07C 29/70* (2006.01) *C08F 4/651* (2006.01)

(86) International application number:
**PCT/CN2011/001536**

(87) International publication number:
**WO 2012/034357 (22.03.2012 Gazette 2012/12)**

## (54) CATALYST CARRIER FOR OLEFIN POLYMERIZATION, SOLID CATALYST COMPONENT AND CATALYST

KATALYSATORTRÄGER FÜR OLEFINPOLYMERISATION, FESTER KATALYSATORBESTANDTEIL UND KATALYSATOR

SUPPORT POUR CATALYSEUR DE POLYMÉRISATION D'OLÉFINES, COMPOSANT CATALYSEUR SOLIDE ET CATALYSEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2010 CN 201010522125**
**16.09.2010 CN 201010283034**

(43) Date of publication of application:
**24.07.2013 Bulletin 2013/30**

(73) Proprietors:
- **China Petroleum & Chemical Corporation**
  **Beijing 100728 (CN)**
- **Beijing Research Institute of Chemical Industry, China Petroleum & Chemical Corporation**
  **Chaoyang District**
  **Beijing 100013 (CN)**

(72) Inventors:
- **TAN, Zhong**
  **Beijing 100013 (CN)**
- **XU, Xiudong**
  **Beijing 100013 (CN)**
- **YAN, Li'an**
  **Beijing 100013 (CN)**
- **ZHOU, Qilong**
  **Beijing 100013 (CN)**
- **SONG, Weiwei**
  **Beijing 100013 (CN)**
- **YIN, Shanshan**
  **Beijing 100013 (CN)**
- **YU, Jinhua**
  **Beijing 100013 (CN)**
- **LI, Fengkui**
  **Beijing 100013 (CN)**
- **WANG, Ying**
  **Beijing 100013 (CN)**
- **REN, Chunhong**
  **Beijing 100013 (CN)**

(74) Representative: **Hoffmann Eitle**
**Patent- und Rechtsanwälte PartmbB**
**Arabellastraße 30**
**81925 München (DE)**

(56) References cited:
**EP-A1- 1 739 070** **CN-A- 1 436 796**
**CN-A- 1 946 664** **CN-A- 101 014 558**
**CN-A- 101 160 329** **CN-A- 101 906 017**
**US-A1- 2005 239 636**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 617 739 B1

## Description

### Technical Field

The present invention relates to a method of preparing a catalyst carrier for olefin polymerization, especially a dialkoxy magnesium carrier.

### Technical Background

Generally, the Ziegler-Natta catalyst including titanium compounds and organo-aluminum compounds is used to obtain olefin polymers in the olefin polymerization field. For example, CN85100997A and CN1453298A disclose that a catalyst comprising a solid catalyst component mainly consisting of titanium, magnesium, chlorine and electron donor compounds, an organo-aluminum compound as a cocatalyst and an organo-silicon compound as an agent for improving stereoregularity, is used in the preparation of polypropylene to obtain propylene polymer. Recently, the researches for catalysts mainly focus on the following aspects, improving the polymerization activity of catalysts, improving the stereoregularity of polyolefins, improving the hydrogen response of catalysts, improving the particle morphology of olefin polymers, reducing the residual chloride in the polymers and so on.

The propylene polymers obtained by the supported catalyst component with dialkoxy magnesium as a carrier possess excellent performances such as excellent particle morphology, a low content of fine powders, good stereoregularity and so on. To obtain such an excellent catalyst component for olefin polymerization, first of all, the dialkoxy magnesium carrier with excellent performances should be prepared.

The known preparation methods for spherical dialkoxyl magnesium mainly contain the following kinds, the first method is to react an alcohol with metal magnesium to prepare dialkoxyl magnesium and then adjust the size of particles by mechanical pulverizing; the second preparation method is to control the final addition ratio of magnesium/ethanol within 9/1 to 1/15 in the reaction of metal magnesium and ethanol, and carry out the reaction intermittently or continuously when ethanol and magnesium are refluxed in ethanol (JP3(1991)-74341); the third preparation method is to spray dry the ethanol solution of magnesium carboxylate after carboxylated, and then make decarboxylation to obtain round fine particles (JP6(1994)-87773); the fourth preparation method is to react metal magnesium with ethanol in the coexistence of saturated hydrocarbons (JP63(1988)-4815).

In the first and second methods, the shapes of particles are pulverized and destroyed. It is very difficult to obtain the particles with well surface morphology and particle size distribution, sometimes at the expense of reducing the output rate. In the third and fourth methods, other raw materials are also needed except Mg and ROH, and the operations are also complex. Each of the above methods has deficiency.

CN1875038A discloses a kind of spherical particles comprising the mixture of hydrocarbyloxy magnesium or hydroxyl magnesium, said spherical particles having a dumping cone highness less than 17mm, and it discloses the use of the mixture of methanol, ethanol and propanol and iodine to prepare spherical particles in the examples. When using such a method to prepare products with a large particle size, the particle size distribution will be wider, and iodine is entirely used as a halogenated agent, and the cost of raw materials is high, which is not beneficial for large-scale industrial application.

There are various researches on preparing an olefin polymerization catalyst component with dialkoxyl magnesium as a carrier. EP0459009 discloses a catalyst component for olefin polymerization. The preparation method is as follows: diethoxy magnesium is dispersed in alkylbenzene to form a suspension; the suspension then touches with titanium tetrachloride and the dichloride of phthaloyl in 80-125°C; after being washed with alkylbenzene, finally the catalyst component containing titanium is obtained. Although the catalyst obtained by such catalyst component has a high activity and a long activity endurance in polymerization, the bulk density of propylene polymers is relatively low.

EP0811639 mainly discloses a solid catalyst component for olefin polymerization, which is prepared by the reaction of a titanium halide, an aryl dicarboxylic diester and an alkoxy magnesium. A solid catalyst component can be obtained by controlling the indexes such as the bulk density of the alkoxy magnesium and the average particle size and controlling the rate of heating the temperature at which the titanium halide first touches with the alkoxy magnesium to the temperature of the reaction (the heating rate is controlled at 0.5 to 20°C /min). Then a polyolefin with high isotacticity and high bulk density can be obtained, but the content of fine powders therein is high.

In CN101054424A, the prepared dialkoxy magnesium is suspended in toluene, titanium tetrachloride is added after the temperature is decreased to 0°C, then the temperature is increased and electron donors are added. Toluene is added to wash the product after full reaction, then titanium compound is used for treating, and finally hexane is used for washing. The polymerization activity of the solid catalyst component is relatively high, the sphericity is good, but the particle size distribution is not concentrated.

## Summary of Invention

The present invention aims to overcome the defects of the prior art and provide a method of preparing a spherical-like dialkoxy magnesium carrier with well performances.

The present invention provides a method of preparing a dialkoxy magnesium carrier as defined in claim 1.

In the above dialkoxy magnesium carrier, the weight ratio of iodine and magnesium chloride is preferably 0.1:1 to 1:0.02. As the chemical activity of magnesium is high, its affinity with oxygen is big, oxide films such as magnesium oxide and magnesium hydroxide will be generated after it is placed in air. When the halogens or halides are added into alcohols, the oxide films on the surface of magnesium can be removed under mild conditions, so that the reaction of alcohol and magnesium can be promoted. In the present invention, only simply mixing the halogenated agents cannot control the particle morphology of dialkoxy magnesium. Through in-depth study, the inventor finds that the reaction rate of alcohols and magnesium can be effectively controlled by selecting the mixed halogenated agents comprising halogens and halides and controlling the ratio of the halogens and the halides, and only when the ratio of the halogens and the halides is appropriate, the particle morphology of dialkoxy magnesium can be maintained. In the mixed halogenated agents, the acidities of the halogens and the halides are that one is strong, and the other is weak. At first, the halogens play the main role in removing the oxide films, and then the halides work together with the pre-added halide, so that the reaction rate can be effectively controlled. At the same time, the price of halogens is much higher than that of halides, the required cost would be lower when the same amounts of halides are used. The mixed halogenated agents can be used in a pure state or a solution state. They can be added into the reaction system respectively, or added after being partially or entirely mixed. The mixed halogenated agents can be the combination of other halogens and halides. The unlimited selection of said halogens and halides can be $I_2$, $Br_2$, $Cl_2$, $MgBr_2$, KI, $MgI_2$, $CaCl_2$, $CaBr_2$, $CaI_2$, $HgCl_2$, $HgBr_2$, $HgI_2$, ethoxy magnesium iodide, methoxy magnesium iodide, isopropyl magnesium iodide, hydrogen chloride, chloroacetyl chloride and so on, wherein, the combination of iodine and magnesium chloride is preferred.

In the above carrier, the molar ratio of said magnesium and the halogen atoms in said mixed halogenated agents is 1:0.0002-1:0.2, preferably 1:0.001-1:0.08. It is found by experiments that the adding amount of halogen atoms will affect the particle morphology and particle size of the final dialkoxy magnesium. When the amount of used halogen atoms is too small, the particle morphology of the obtained dialkoxy magnesium is poor, and if the dosage of halogen atoms is too big, not only the cost for preparing dialkoxy magnesium will increase, but also the particle size of dialkoxy magnesium will be uneven, and the reaction is difficult to control.

In said dialkoxy magnesium carrier, the weight ratio of alcohols and magnesium is 4:1-50:1, preferably 6:1-25:1. Said alcohols are mixed alcohols, which can better adjust the reaction rate and control the reaction process. The kinds and ratio of mixed alcohols can be changed according to the requirements of the product structures. Ethanol is the main component and the other alcohols are the minor components in the mixed alcohols. There is no specific limitation on the content of water in the alcohols used in the present invention, but in order to make the obtained dialkoxy magnesium with better performances, the content of water is required to be as low as possible. Generally, the content of water in the alcohols is controlled at no more than 1000ppm, preferably no more than 200ppm.

Said alcohols comprise ethanol and at least one selected from $C_6$-$C_{11}$ alcohols, comprising n-heptanol, 2-ethyl hexanol, n-hexanol, n-heptanol, n-octanol, n-nonanol, n-decanol, 2-hexanol, 2-heptanol, 2-octanol, 2-nonanol, 2-decanol, 2-ethyl butanol, 2-ethyl hexanol, 4-methyl-2-pentanol, 3,3,5-trimethyl pentanol, 4-methyl-3-heptanol, benzyl alcohol, 2-phenyl ethanol, 1-phenyl-1-propanol and so on, preferably ethanol and at least one selected from $C_6$-$C_8$ alcohols, more preferably ethanol and 2-ethyl hexanol, and in the finally obtained carrier, the content of ethoxy magnesium is equal to or higher than 80wt%, and the content of 2-ethyl hexyloxy magnesium is 0.01 to 20wt%.

Said alcohols further comprise ethanol and at least one selected from $C_1$-$C_5$ lower alcohols, including not ethanol but rather methanol, n-propanol, n-butanol, n-pentanol, 2-propanol, 2-butanol, 2-pentanol, ethylene glycol, glycerin and so on, preferably ethanol and isopropanol, when the content of ethoxy magnesium is equal to or higher than 80wt%, and the content of isopropoxy magnesium is 0.01 to 20wt% in the obtained carrier.

Said alcohols preferably comprise ethanol, at least one selected from $C_6$-$C_{11}$ alcohols and at least one selected from $C_1$-$C_5$ lower alcohols, not including ethanol. More preferably, said alcohols comprise ethanol, 2-ethyl hexanol and isopropanol. The three alcohols can be added at the same time, or added respectively in a certain order, on which there is no specific limitation. In the finally obtained carrier, the content of ethoxy magnesium is equal to or higher than 80wt%, the content of 2-ethyl hexyloxy magnesium is 0.01 to 19.9wt% and the content of isopropoxy magnesium is 0.01 to 19.9wt%.

Said dialkoxy magnesium carrier is spherical-like, and the average particle size is 5 to 150$\mu$m, preferably 8~150$\mu$m, further preferably 8 to 100$\mu$m. In a specific example, the average particle size is 10 to 80$\mu$m. The particle size distribution index SPAN is less than 1.1, preferably less than 1.05, wherein the calculation formula of SPAN is as follows,

$$SPAN = (D_{90}-D_{10})/D_{50}$$

In the formula, $D_{90}$ shows the particle size corresponding to 90% cumulative weight fraction, $D_{10}$ shows the particle size corresponding to 10% cumulative weight fraction, and $D_{50}$ shows the particle size corresponding to 50% cumulative weight fraction.

In the present invention, the used magnesium is magnesium metal. As long as its reaction performance is good, it can be in any shapes, including granular, ribbon shaped or powdered ones. In order to make the average particle size of the generated dialkoxy magnesium within a suitable range and the particle morphology to be good, the preferable method is to require the magnesium metal to be spherical particles with an average size of 10-360μm, more preferably 50~300μm, which can maintain the reaction performance to be relatively uniform.

In addition, as the chemical activity of magnesium is strong, the oxide films such as magnesium oxide and magnesium hydroxide are generally formed on the surface of magnesium, but there is no specific limitation on the oxide films on the surface of magnesium metal.

Said inert atmosphere in the present invention comprises nitrogen atmosphere, argon atmosphere and so on, preferably nitrogen atmosphere.

The preparation method of said dialkoxyl magnesium carrier comprises the following steps: magnesium and said alcohols are used as raw materials, and the reflux reaction is carried out in the presence of said mixed halogenated agents to prepare spherical-like particle dialkoxy magnesium under an inert atmosphere.

An inert organic solvent can be used in the preparation process according to the situation. In the present invention, said inert solvent can be selected from at least one of $C_6$-$C_{10}$ alkanes or aromatics, preferably selected from at least one of hexane, heptanes, octane, decane, benzene, toluene, xylene or their derivations, and so on.

In the above preparation process, the adding order of each reactant can be determined according to actual needs. Specifically, there is no particularly limitation on the adding method of halogenated agents. It can be added after being dissolved in alcohols or directly added into the magnesium and alcohols in the solid state or the liquid state; the method of adding the alcohol solution of halogenated agents dropwise when heating the solution of magnesium metal and alcohol can also be used, after which the reaction for preparing carrier can be carried out.

As to the addition of magnesium, alcohols, halogenated agents and the inert solvent, they can be added in one time or successively. Adding the raw materials successively can prevent the generation of a large amount of hydrogen instantaneously and the droplet of alcohols or halogenated agents resulted from the generation of a large amount of hydrogen instantaneously, so such feeding method is preferred when taking the security and reaction uniformity into account. The number of feeding times can be determined by the reactor scale and dosage of each material.

Said reaction is carried out between 0°C and the reflux temperature of the reaction system in the above preparation steps for the dialkoxy magnesium carrier. The minor changes of reaction pressure will change the reflux temperature. The higher the reaction temperature is, the more quickly the reaction is carried out. The reaction temperature during the reaction can also be changed. The particle size and particle morphology can be changed by selecting different reaction temperatures. Said reaction time is 2-30h. In the actual operation, when the emission of hydrogen stops, the reaction can be judged as having stopped accordingly.

After the completion of reaction, the obtained final product can be saved after being dried or by being suspended in the inert diluents used for preparing the solid catalyst component in the next step.

The dialkoxy magnesium carrier produced by the method of the present invention can be used for a solid catalyst component for olefin polymerization, wherein said catalyst component comprises the reaction products of the following components in an inert solvent,

a) said dialkoxy magnesium carrier;

b) titanium compounds, selected from at least one of the compounds as shown in Formula $Ti(OR)_{4-n}X_n$, wherein R is selected from $C_1$-$C_{14}$ aliphatic hydrocarbyl or aromatic hydrocarbyl groups, X is a halogen atom, and n is an integer selected from 0 to 4. When n is equal to or higher than 2, the existing plurality of Rs can be same with or different form each other;

c) electron donor compounds, selected from $C_1$-$C_4$ alkyl esters of saturated aliphatic carboxylic acids, $C_7$-$C_8$ alkyl esters of aromatic carboxylic acids, $C_2$-$C_6$ aliphatic ethers, $C_3$-$C_4$ cycloethers, $C_3$-$C_6$ saturated aliphatic ketones and/or diol ester compounds selected from those as shown in Formula (I);

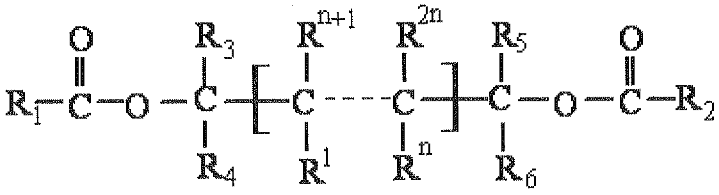

(I)

wherein $R_1$-$R_6$ and $R^1$-$R^{2n}$ groups, the same with or different from each other, can be hydrogen atom, halogen atom, substituted or unsubstituted straight chain or branched chain $C_1$-$C_{20}$ alkyl, $C_3$-$C_{20}$ cycloalkyl, $C_6$-$C_{20}$ aryl, $C_7$-$C_{20}$ alkaryl, $C_7$-$C_{20}$ aralkyl, $C_2$-$C_{10}$alkenyl, $C_{10}$-$C_{20}$ fused ring aryl or ester group; $R_1$ and $R_2$ are not hydrogen atoms; $R_3$-$R_6$ and $R^1$-$R^{2n}$ groups can optionally comprise one or more heteroatoms as the substituent of carbon and/or hydrogen atoms, said heteroatoms selected from nitrogen, oxygen, sulfur, silicon, phosphorus and halogen atoms; one or more of $R_3$-$R_6$ and $R^1$-$R^{2n}$ groups can optionally form a ring with each other; n is an integer selected from 0 to 10.

The electron donor compounds as shown in Formula (I) preferably comprises the compounds as shown in Formula (II)

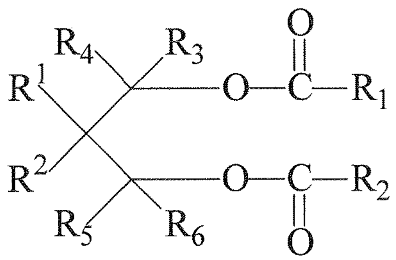

(II)

wherein $R_1$-$R_6$ and $R^1$-$R^2$ groups, the same with or different from each other, can be hydrogen atom, halogen atom, straight chain or branched chain $C_1$-$C_{20}$ alkyl, $C_3$-$C_{20}$ cycloalkyl, $C_6$-$C_{20}$ aryl, $C_7$-$C_{20}$ alkaryl or $C_7$-$C_{20}$ aralkyl group.

In the above solid catalyst component, the dosage of titanium compounds is 0.5 to 100mol, preferably 1 to 50mol, based on per molar of dialkoxy magnesium compounds.

In the Formula of said titanium compounds in the above solid catalyst component, X is preferably the chlorine atom or the bromine atom, more preferably the chlorine atom; the structure of R has many possibilities, comprising saturated or unsaturated group, straight chain group or branched chain group or cyclic chain group. The preferable $C_1$-$C_{14}$ group can be alkyl, alkenyl, cycloalkenyl or aralkyl group, especially $C_1$-$C_{14}$ straight chain or branched chain alkyl group. When n is equal to or higher than 2, the existing plurality of Rs can be the same with or different form each other. The specific examples of R are selected from at least one of methyl, ethyl, n-propyl, isopropyl, allyl, n-butyl, sec-butyl, isobutyl, n-pentyl, n-hexyl, n-heptyl, n-octyl, n-decyl, allyl, butenyl, cyclopentyl, cyclohexyl, cyclohexenyl, benzyl, phenyl, tolyl and phenylethyl groups. Said titanium compounds are selected from at least one of tetraalkoxy titanium, titanium tetrahalide, alkoxytitanium trihalide, dialkoxy titanium dihalide and trialkoxy titanium halide. More specifically, said tetraalkoxy titanium is selected from at least one of tetramethoxy titanium, tetraethoxy titanium, tetrapropoxy titanium, tetra-iso-propoxy titanium, tetra-n-butoxy titanium, tetra-iso-butoxy titanium, tetracyclohexyloxy titanium and tetraphenoxy titanium. Said titanium tetrachloride is selected from at least one of titanium tetrachloride, titanium tetrabromide and titanium tetraiodide. Said alkoxy titanium trichloride is selected from at least one of methoxy titanium trichloride, ethoxy titanium trichloride, propoxy titanium trichloride, n-butoxy titanium trichloride and ethoxy titanium tribromide. Said dialkoxy titanium dichloride is selected from at least one of dimethoxy titanium dichloride, diethoxy titanium dichloride, di-n-propoxy titanium dichloride, di-iso-propoxy titanium dichloride and diethoxy titanium dibromide. Said trialkoxy titanium chloride is selected from at least one of trimethoxy titanium chloride, triethoxy titanium chloride, tri-n-propoxy titanium chloride, tri-iso-propoxy titanium chloride. Titanium tetrahalide is preferred, and titanium tetrachloride is particularly preferred.

In the above solid catalyst component, the dosage of electron donor compounds is generally 0.005 to 10mol, preferably 0.01 to 1mol, based on per molar of dialkoxy magnesium compounds. Said electron donor compounds can be used in combination with one another.

In the above catalyst component, the specific examples of $C_1$-$C_4$ alkyl esters of saturated aliphatic carboxylic acids, $C_7$-$C_8$ alkyl esters of aromatic carboxylic acids, $C_2$-$C_6$ aliphatic ethers, $C_3$-$C_4$ cycloethers, $C_3$-$C_6$ saturated aliphatic ketones comprise methyl formate, ethyl acetate, butyl acetate, diisobutyl phthalate, din-butyl phthalate, diisooctyl phthalate, 1,3-diamyl phthalate, ethyl ether, hexyl ether, tetrahydrogen furan (THF), acetone, methyl iso-butyl ketone and so on, preferably at least one of din-butyl phthalate, diisobutyl phthalate and 1,3-diamyl phthalate.

In the above solid catalyst component, said diol ester compound electron donors are disclosed in CN1436766 and CN1436796A. The related disclosures in the above patents are totally introduced into the present invention as a reference. Said diol ester compounds as shown in Formula (II) comprise 2-ethyl-1,3-glycol dibenzoate, 2-propyl-1,3-glycol dibenzoate, 2-isopropyl-2-isoamyl-1,3-glycol dibenzoate, 1,3-butanediol dimethyl benzoate, 2-methyl-1,3-butanediol dim-chlorobenzoate, 2,3-dimethyl-1,3-butanediol dibenzoate, 1,3-pentanediol pivalate, 2,4-pentanediol dibenzoate, 2-methyl-1,3-pentanediol benzoic cinnamate, 2,2-dimethyl-1,3-pentanediol dibenzoate, 2,4-heptanediol dibenzoate, 2-methyl-3,5-heptanediol dibenzoate and so on, preferably at least one of 2,4-pentanediol dibenzoate and 4-ethyl-3,5-heptanediol dibenzoate.

In the above solid catalyst component, the inert solvent is used to disperse and dilute the materials so as to make the liquids to react under a good stirring state, and it can also eliminate partial static electricity and make a certain effect on maintaining well particle morphology. The dosage of said inert diluents is 0.5 to 100mol, preferably 1 to 50mol, based on per molar of dialkoxy magnesium compounds. The inert solvent herein is preferably toluene.

The above solid catalyst component for olefin polymerization using spherical-like dialkoxy magnesium as a carrier can be prepared by the following steps. The above dialkoxy magnesium compounds react with titanium compounds and electron donor compounds in the presence of an inert solvent to obtain the solid, and then the above solid is washed with an inert solvent to obtain the solid catalyst component.

In the above method, said dialkoxy magnesium, titanium compound, inert solvent and electron donor compound are preferably to react on the following conditions. Said reaction temperature is generally -40 to 200°C, preferably -20 to 150°C; said reaction time is generally 1min to 20h, preferably 5min to 8h.

In the above method, there is no specific limitation on the addition order of each reactant. For example, each component can mix with one another in the presence of the inert solvent, or each component can be previously diluted by the inert solvent before the mixing. There is no specific limitation on the number of times of mixing, which can be once or many times.

The inert solvent for the above washing is preferably hexane. There is no specific limitation on the washing method, but decantation, filtration and so on are preferred. There is no specific limitation on the dosage of the inert solvent, the washing time and the number of washing times. Based on per molar of dialkoxy magnesium compounds, 1 to 1000mol, preferably 10 to 500 mol of the solvent is generally used to wash for 1 to 24h, preferably 6 to 10h. In addition, it is preferred to stir under washing operation from the point of washing uniformity and washing efficiency.

Said solid catalyst component can be saved in dry condition or in an inert solvent.

The above solid catalyst component can be used for a catalyst for olefin polymerization comprising

a) said solid catalyst component;

b) organo-aluminum compound, selected from at least one of the compounds as shown in Fornula $AlR_nX_{3-n}$, wherein R is selected from hydrogen atoms and $C_1$-$C_{20}$ hydrocarbyl groups, X is the halogen atom, and n is an integer selected from higher than 0 to 3;

c) optional organo-silicon compound, whose formula is $R_1R_2Si(OR')_2$, wherein $R_1$ and $R_2$, the same with or different from each other, can be $C_1$-$C_{20}$ alkyl, cycloalkyl or aryl group, and R' is $C_1$-$C_4$ alkyl group.

In the above catalyst for olefin polymerization, the specific examples of said organoaluminum compound comprise alkyl aluminum halides, such as trimethyl aluminum, triethyl aluminum, triisobutyl aluminum, trioctyl aluminum, diethyl aluminum chloride, diisobutyl aluminum chloride, ethyl aluminum dichloride and so on preferably triethyl aluminum or triisobutyl aluminum. The ratio of said organoaluminum compounds and said solid catalyst component is 5-5000:1, preferably 20-500:1, based on the molar ratio of aluminum and titanium.

In the above catalyst for olefin polymerization, the ratio of said organosilicon compound and organoaluminum compound is 2-100:1, preferably 5-50:1, based on the molar ratio of aluminum and silicon. The specific examples of said organosilicon compound comprise trimethyl methoxy silane, trimethyl ethoxy silane, trimethyl phenoxy silane, dimethyl dimethoxy silane, dimethyl diethoxy silane, cyclohexyl methyl diethoxy silane, methyl cyclohexyl dimethoxy silane, diphenyl dimethoxy silane, diphenyl diethoxy silane, phenyl triethoxy silane, phenyl trimethoxy silane, vinyl trimethoxy silane and so on, preferably cyclohexyl methyl dimethoxy silane or diisopropyl dimethoxy silane.

By using the above provided carrier, solid catalyst component and catalyst, the catalyst in the present invention can be used for olefin polymerization, comprising homopolymerization and copolymerization, said olefin containing propylene, ethylene and so on, whose polymerization activity is high and through which olefin polymers with stereoregularity and good particle morphology can be obtained. Ethylene-propylene copolymers with high ethylene content can

be prepared according to the present invention. As the stereoregularity is good when said catalyst is used in propylene homopolymerization, the prepared copolymers are suitable to be used as the raw material for impact resistant products with balanced physical properties such as rigidness and impact resistance.

The method of the present invention provides a spherical-like dialkoxy magnesium carrier with good performances. Iodine and magnesium chloride are used as halogenated agents. The dosages of halogenated agents and mixed alcohols are small, and the preparation cost of dialkoxy magnesium is low. Said dialkoxy magnesium has good morphology, and its particle size distribution is even with small span. When the catalyst prepared by using such carrier is used for polymerization, the activity is high, and the obtained polymers have high bulk density, high isotacticity, good particle morphology, even distribution and low content of fine powders.

## Description of Drawing

Figure 1 The photomicrograph of the dialkoxy magnesium carrier according to a example of the present invention;
Figure 2 The photomicrograph of the carrier according to comparative example 1 of the present invention.

## Embodiment

The present invention will be explained in detail by the following examples.

It should be noted that, the evaluation of magnesium compounds and polyolefins prepared in the examples are made by the following methods.

(1) The content of titanium in the catalyst is tested by 721 spectrophotometer;
(2) The isotactity (II) of the polymers is tested by the boiling n-heptane extraction method;
(3) The melt index of the polymers (MI) is tested according to testing standards GB/T3682-2000;
(4) The particle size and particle size distribution of the dialkoxy magnesium and the catalysts are tested by Malvern Mastersizer TM2000 laser diffraction method with n-hexane as a dispersant, wherein SPAN= $(D_{90}-D_{10})/D_{50}$;
(5) The bulk density (BD) is tested by the method of the weight of loose solid in each unit volume;
(6) The content of ethylene is tested by $^{13}C$-NMR;
(7) The content of insoluble component in xylene (XS%) at room temperature is tested according to ISO 16152-2005;
(8) The content of the alkoxy group is tested by first acidolysis of the alkoxy magnesium carrier, then neutralizing the excess acid, and finally using the chromatography for test.

### Example 1

(1) The preparation of dialkoxy magnesium carrier

After the air in 16L pressure-resistant reactor is fully replaced with nitrogen, 10000ml of ethanol, 300ml of 2-ethyl hexanol and 200ml of isopropanol are added into the reactor, and 12g of iodine and 8g of magnesium chloride are also added to be dissolved. The temperature is increased after stirring to reach the reflux temperature of the reaction system. Then a total of 640g of magnesium powders are successively added. The reaction is carried out until the reaction is completed, i.e. no hydrogen is discharged. Then the dialkoxy magnesium carrier 1# is obtained after being washed, separated and dried. The data are shown in Table 1. The photomicrograph is shown in Figure 1.

(2)The preparation of the catalyst component

650g of the above dialkoxy magnesium carrier 1#, 3250ml of toluene and 65ml of din-butyl phthalate (DNBP) are used to make to a suspension. 2600ml of toluene and 3900ml of titanium tetrachloride are added into a 16L pressure-resistant reactor after the gas therein is repeatedly replaced with high purity nitrogen. Then the temperature is decreased to -5°C, after which the above prepared suspension is added into the reactor. After the temperature is maintained for 1h, the temperature is slowly increased up to 110°C, and 65ml of DNBP is added when the temperature is increased to 80°C. The temperature is maintained at 110°C for 2h, and the liquid is removed by filtration under pressure. Then the mixed solution of 5070ml of toluene and 3380ml of titanium tetrachloride is added, and said mixed solution is used to treat the solid for 1h under stirring when the temperature is increased to 110°C. Such treatment is repeated for 3 times before the liquid is removed by filtration. The obtained solid is washed with 150ml of hexane for 4 times, then the liquid is removed by filtration, and then the solid catalyst component is obtained after being dried. The data are shown in Table 2.

(3) The olefin polymerization

Propylene is used as said olefin, and is polymerized according to the following steps:
Into a 5L steel autoclave equipped with a stirrer, after the air therein is replaced with nitrogen, 5ml of hexane solution of triethyl aluminium (the concentration of triethyl aluminium is 0.5mmol/ml), 1ml of hexane solution of cyclohexyl methyl dimethoxy silane (CHMMS) (the concentration of cyclohexyl methyl dimethoxy silane is 0.10mmol/ml), 10ml of anhydrous hexane and 10mg of the solid catalyst component are introduced in nitrogen stream at room temperature. After the autoclave is closed, 4.5Lof hydrogen (in the standard state) and 2.0L of liquid propylene are introduced. Then the temperature is increased to 70°C in 10 min under stirring. After the polymerization is carried out at 70°C for 1h, the stirring is stopped, the unpolymerized propylene monomers are removed and the polymers are collected. The data are shown in Table 2.

**Example 2**

Except that 10200ml of ethanol is added in the preparation of a dialkoxy magnesium carrier, and isopropanol is not added, the other conditions are similar to those in Example 1. The data are shown in Tables 1 and 2.

**Example 3**

Except that 10300ml of ethanol is added in the preparation of the dialkoxy magnesium carrier, and 2-ethyl hexanol is not added, the other conditions are similar to those in Example 1. The data are shown in Tables 1 and 2.

**Example 4**

Except that 10400ml of ethanol and 100ml of 2-ethyl hexanol are added in the preparation of the dialkoxy magnesium carrier, the other conditions are similar to those in Example 2. The data are shown in Tables 1 and 2.

**Example 5**

Except that 10000ml of ethanol and 500ml of 2-ethyl hexanol are added in the preparation of the dialkoxy magnesium carrier, the other conditions are similar to those in Example 2. The data are shown in Tables 1 and 2.

**Example 6**

Except that 6g of iodine and 4g of magnesium chloride are added in the preparation of a dialkoxy magnesium carrier, the other conditions are similar to those in Example 2. The data are shown in Tables 1 and 2.

**Example 7**

Except that 2g of iodine and 3g of magnesium chloride are added in the preparation of the dialkoxy magnesium carrier, the other conditions are similar to those in Example 2. The data are shown in Table 1 and 2.

**Example 8**

Except that the adding amount of ethanol, 2-ethyl hexanol and isopropanol in the preparation of the dialkoxy magnesium carrier are adjusted 10200ml, 150ml and 150ml respectively, the other conditions are similar to those in Example 2. The data are shown in Tables 1 and 2.

**Example 9**

Except that the 2-ethyl of hexanol is replaced with n-heptanol in the preparation of the dialkoxy magnesium carrier, the other conditions are similar to those in Example 2. The data are shown in Tables 1 and 2.

**Example 10**

Except that the isopropanol is replaced with n-butanol in the preparation of the dialkoxy magnesium carrier, the other conditions are similar to those in Example 3. The data are shown in Tables 1 and 2.

**Example 11**

(1) The preparation of the dialkoxy magnesium carrier

It is similar to Example 1. The data are shown in Table 1.

(2) The preparation of the catalyst component

650g of the above-mentioned dialkoxy magnesium carrier and 3250ml of toluene are used to make to a suspension. 2600ml of toluene and 3900ml of titanium tetrachloride are added into a 16L pressure-resistant reactor, after the air therein is repeatedly replaced with high purity nitrogen. After the temperature is decreased to -5°C, the above prepared suspension is added into the reactor. After the temperature is maintained for 1h, the temperature is slowly increased up to 110°C, and 65ml of DNBP is added when the temperature is increased to 80°C. The temperature is maintained at 110°C for 2h, and the liquid is removed by filtration under pressure. Then the mixed solution of 5070ml of toluene and 3380ml of titanium tetrachloride is added, and said mixed solution is used to treat the solid for 1h under stirring when the temperature is increased to 110°C. Such treatment is repeated for 3 times, and the liquid is removed by filtration. The obtained solid is washed with 150ml of hexane for 4 times, the liquid is removed by filtration, and the solid catalyst component is obtained after being dried. The data are shown in Table 2.

(3) The olefin polymerization

The steps are similar to those in Example 1. The data are shown in Table 2.

**Example 12**

(1) The preparation of the dialkoxy magnesium carrier

It is similar to Example 1. The data are shown in Table 1.

(2)The preparation of the catalyst component

650g of the above-mentioned dialkoxy magnesium carrier, 3250ml of toluene and 182ml of din-butyl phthalate (DNBP) are used to make to a suspension. 2800ml of toluene and 3900ml of titanium tetrachloride are added into a 16L pressure-resistant reactor after the air therein is repeatedly replaced with high purity nitrogen. After the temperature is decreased to -5°C, the above prepared suspension is added into the reactor. After the temperature is maintained for 1h, the temperature is slowly increased up to 110°C, and 130ml of DNBP is added when the temperature is increased to 80°C. The temperature is maintained at 110°C for 2h, and the liquid is removed by filtration under pressure. Then the mixed solution of 5070ml of toluene and 3380ml of titanium tetrachloride is added, and said mixed solution is used to treat the solid for 1h under stirring when the temperature is increased to 110°C. Such treatment is repeated for 3 times, and the liquid is removed by filtration. The obtained solid is washed with 150ml of hexane for 4 times, then the liquid is removed by filtration, and then the solid catalyst component is obtained after being dried. The data are shown in Table 2.

(3) The olefin polymerization

The step is similar to Example 1. The data are shown in Table 2.

**Comparative Example 1**

(1) The preparation of the dialkoxy magnesium carrier

After the air in a 16L pressure-resistant reactor is fully replaced with nitrogen, 13300ml of ethanol is added into the reactor, and 23g of iodine is also added. The temperature is increased after stirring to reach the reflux temperature of the reaction system. Then a total of 640g of magnesium powders are successively added. The reaction is carried out until the reaction is completed, i.e. no hydrogen is discharged. Then the dialkoxy magnesium carrier is obtained after being washed, separated and dried. The data are shown in Table 1. The photomicrograph is shown in Figure 2.

(2)The preparation of the catalyst component

Except that the dialkoxy magnesium carrier in the above step (1) is used, the others are similar to those in Example 1. The data are shown in Table 2.

(3) The olefin polymerization

The steps are similar to those in Example 1. The data are shown in Table 2.

**Example 13**

(1) The preparation of the dialkoxy magnesium carrier

A 1L reactor with a stirrer is equipped with a reflux condenser, a thermometer and a burette. After the air in the reactor is fully replaced with nitrogen, 550ml of ethanol and 10ml of isopropanol are added into the reactor, and 0.68g of iodine and 0.42g of magnesium chloride are also added to be dissolved. The temperature is increased after stirring to reach the reflux temperature of the reaction system. Then a total of 32g of magnesium powders are successively added, and 90ml of toluene is introduced. The reaction is carried out until the reaction is completed, i.e. no hydrogen is discharged. Then dialkoxy magnesium carrier is obtained after being washed, separated and dried. The data are shown in Table 1.

(2)The preparation of the catalyst component

10g of the above dialkoxy magnesium carrier, 50ml of toluene and 2.0ml of 4-ethyl-3, 5-heptanediol dibenzoate are used to make a suspension. 10ml of toluene and 90ml of titanium tetrachloride are added into the 300ml reactor after the air therein is repeatedly replaced with high purity nitrogen. After the temperature is increased to 80°C, the above prepared suspension is added into the reactor. After the temperature is maintained for 1h, the temperature is slowly increased up to 115°C and maintained for 2h, and the liquid is removed by filtration under pressure. Then the mixed solution of 120ml of toluene and 30ml of titanium tetrachloride is added, and said mixed solution is used to treat the solid for 1h under stirring when the temperature is increased to 110°C. Such treatment is repeated for 3 times, and the liquid is removed by filtration. The obtained solid is washed with 150ml of hexane for 4 times, the liquid is removed by filtration, and the solid catalyst component is obtained after being dried. The data are shown in Table 2.

(3) The olefin polymerization

The steps are similar to those in Example 1. The data are shown in Table 2.

**Example 14**

Except that the 4-ethyl-3, 5-heptanediol dibenzoate is replaced by 3,5-heptanediol dibenzoate in the preparation of the catalyst component, the others are similar to those in Example 13. The data are shown in Table 2.

**Example 15**

Except that the 4-ethyl-3, 5-heptanediol dibenzoate is replaced by 2,4-pentanediol dibenzoate in the preparation of the catalyst component, the others are similar to those in Example 13. The data are shown in Table 2.

**Example 16**

(1) The preparation of the dialkoxy magnesium carrier

It is similar to Example 13.

(2) The preparation of the catalyst component

10ml of toluene and 90ml of titanium tetrachloride are added into a 300ml reactor after the air therein is repeatedly replaced with high purity nitrogen. The temperature is decreased to -5°C, and then the prepared suspension of 10g of dialkoxy magnesium and 50ml of toluene in Example 13 is added. After the temperature is slowly increased to 80°C,

1.8ml of 4-ethyl-3, 5-heptanediol dibenzoate is added. The temperature is increased up to 115°C and maintained for 2h, and the liquid is removed by filtration under pressure. Then the mixed solution of 120ml of titanium tetrachloride and 30ml of toluene is added, and said mixed solution is used to treat the solid for 1h under stirring when the temperature is increased to 110°C. Such treatment is repeated for 3 times, and the liquid is removed by filtration. The obtained solid is washed with 150ml of hexane for 4 times, the liquid is removed by filtration, and the solid catalyst component is obtained after being dried.

(3) The olefin polymerization

The steps are similar to those in Example 13. The data are shown in Table 2.

**Example 17**

Except that the 4-ethyl-3, 5-heptanediol dibenzoate is replaced by 3,5-heptanediol dibenzoate in the preparation of the catalyst component, the others are similar to those in Example 16. The data are shown in Table 2.

**Example 18**

Except that the 4-ethyl-3, 5-heptanediol dibenzoate is replaced by 2,4-pentanediol dibenzoate in the preparation of the catalyst component, the others are similar to those in Example 13. The data are shown in Table 2.

**Example 19**

(1) The preparation of the dialkoxy magnesium carrier

A 1L reactor with a stirrer is equipped with a reflux condenser, a thermometer and a burette. After the air in the reactor is fully replaced with nitrogen, 550ml of ethanol and 10ml of isopropanol are added into the reactor, and 0.08g of iodine and 0.8g of magnesium chloride are also added to be dissolved. The temperature is increased after stirring to reach the reflux temperature of the reaction system. Then a total of 32g of magnesium powders are successively added, and 90ml of toluene is introduced. The reaction is carried out until the reaction is completed, i.e. no hydrogen is discharged. Then the dialkoxy magnesium carrier is obtained after being washed, separated and dried. The data are shown in Table 1.

(2) The preparation of the catalyst component

The steps are similar to those in Example 13.

(3) The olefin polymerization

The steps are similar to those in Example 13. The data are shown in Table 2.

**Example 20**

(1) The preparation of the dialkoxy magnesium carrier

A 1L reactor with a stirrer is equipped with a reflux condenser, a thermometer and a burette. After the air in the reactor is fully replaced with nitrogen, 550ml of ethanol and 10ml of isopropanol are added into the reactor, and 2g of iodine and 0.04g of magnesium chloride are also added to be dissolved. The temperature is increased after stirring to reach the reflux temperature of the reaction system. Then a total of 32g of magnesium powders are successively added, and 90ml of toluene is introduced. The reaction is carried out until the reaction is completed, i.e. no hydrogen is discharged. Then the dialkoxy magnesium carrier is obtained after being washed, separated and dried. The data are shown in Table 1.

(2) The preparation of the catalyst component

The steps are similar to those in Example 13.

(3) The olefin polymerization

The steps are similar to those in Example 13. The data are shown in Table 2.

**Example 21**

(1) The preparation of the dialkoxy magnesium carrier

A 1L reactor with a stirrer is equipped with a reflux condenser, a thermometer and a burette. After the air in the reactor is fully replaced with nitrogen, 560ml of ethanol and 50ml of 2-ethyl hexanol are added into the reactor, and 2g of iodine and 0.2g of magnesium chloride are also added to be dissolved. The temperature is increased after stirring to reach the reflux temperature of the reaction system. Then a total of 32g of magnesium powders are successively added, and 30ml of toluene is introduced. The reaction is carried out until the reaction is completed, i.e. no hydrogen is discharged. Then the dialkoxy magnesium carrier is obtained after being washed, separated and dried. The data are shown in Table 1.

(2) The preparation of the catalyst component

The steps are similar to those in Example 1.

(3) The olefin polymerization

The steps are similar to those in Example 1. The data are shown in Table 2.

**Comparative Example 2**

(1) The preparation of the dialkoxy magnesium carrier

It is similar to Example 13.

(2) The preparation of the catalyst component

10g of the above dialkoxy magnesium carrier, 50ml of toluene and 2.5ml of din-butyl phthalate (DNBP) are used to make to s suspension. 10ml of toluene and 90ml of titanium tetrachloride are added into a 300ml reactor after the air therein is repeatedly replaced with high purity nitrogen. After the temperature is increased to 80°C, the above prepared suspension is added into the reactor. After the temperature is maintained for 1h, the temperature is slowly increased up to 115°C and maintained for 2h, and the liquid is removed by filtration under pressure. Then the mixed solution of 120ml of toluene and 30ml of titanium tetrachloride is added, and said mixed solution is used to treat the solid for 1h under stirring when the temperature is increased to 110°C. Such treatment is repeated for 3 times, and the liquid is removed by filtration. The obtained solid is washed with 150ml hexane for 4 times, the liquid is removed by filtration, and the solid catalyst component is obtained after being dried. The data are shown in Table 2.

(3) The olefin polymerization

The steps are similar to those in Example 13. The data are shown in Table 2.

**Comparative Example 3**

(1) The preparation of the catalyst component

Into a reactor in which the gas is repeatedly replaced with high purity nitrogen, 4.8g of anhydrous magnesium chloride, 100ml of toluene, 4.0ml of epichlorohydrin and 12.5ml of tributyl phosphate are added. The reaction mixture is made to react for 2h with a stirring speed of 450rpm and a temperature of 60°C. 1.4g of phthalic anhydride is added, and after the reaction is continued for 1h, the temperature is decreased to -28°C. 56ml of titanium tetrachloride is added and the temperate is increased slowly to 85°C, and 2.0ml of 2, 4-pentanediol dibenzoate is added at 80°C. When the temperature reaches 85°C, it is kept for 1h. After the mother liquor is removed by filtration, the residual solid is first washed with 0.95mol of toluene for 2 times, and then treated with 0.57mol of toluene and 0.36mol of titanium tetrachloride for 2h at 110°C. Such treatment is repeated once again after filtration. The obtained solid is washed with hexane for 5 times and dried, and the solid titanium-containing catalyst component is obtained.

(2) The olefin polymerization

The steps are similar to those in Example 13. The data are shown in Table2.

Table 1 The data of dialkoxy magnesium carrier

| | Halogenated agents | $I_2$: $MgCl_2$ wt:wt | Halogen atom/magnesium mol/mol | Alcohols/ magnesium wt/wt | Ethanol ml | 2-ethyl hexanol ml | Isopropanol ml | Average particle size $D_{50}$ um | toluene ml | Particle size distribution SPAN | Bulk density g/cm$^3$ | Alkoxy magnesium wt% | |
|---|---|---|---|---|---|---|---|---|---|---|---|---|---|
| | | | | | | | | | | | | 2-ethyl hexyloxy magnesium | isopropoxy magnesium |
| Example 1 | $I_2$, $MgCl_2$ | 1.5:1 | 0.01:1 | 13.0:1 | 10000 | 300 | 200 | 79.6 | 0 | 0.67 | 0.39 | 1.5 | 1.2 |
| Example 2 | $I_2$, $MgCl_2$ | 1.5:1 | 0.01:1 | 13.0:1 | 10000 | 300 | 0 | 76.8 | 0 | 0.68 | 0.37 | 1.8 | 0 |
| Example 3 | $I_2$, $MgCl_2$ | 1.5:1 | 0.01:1 | 12.8:1 | 10000 | 0 | 200 | 69.3 | 0 | 0.73 | 0.35 | 0 | 1.5 |
| Example 4 | $I_2$, $MgCl_2$ | 1.5:1 | 0.01:1 | 13.0:1 | 10000 | 100 | 0 | 65.2 | 0 | 0.77 | 0.35 | 0.7 | 0 |
| Example 5 | $I_2$, $MgCl_2$ | 1.5:1 | 0.01:1 | 13.0:1 | 10000 | 500 | 0 | 82.5 | 0 | 0.63 | 0.38 | 3.8 | 0 |
| Example 6 | $I_2$, $MgCl_2$ | 1.5:1 | 0.005:1 | 13.0:1 | 10000 | 300 | 0 | 39.6 | 0 | 0.82 | 0.37 | 1.7 | 0 |
| Example 7 | $I_2$, $MgCl_2$ | 1:1.5 | 0.003:1 | 13.0:1 | 10000 | 300 | 200 | 25.8 | 0 | 0.81 | 0.28 | 1.6 | 1.1 |
| Example 8 | $I_2$, $MgCl_2$ | 1.5:1 | 0.01:1 | 13.0:1 | 10200 | 150 | 150 | 72.2 | 0 | 0.75 | 0.36 | 1.0 | 1.2 |
| Example 9 | $I_2$, $MgCl_2$ | 1.5:1 | 0.01:1 | 12.8:1 | 10000 | n-heptanol 300 | 0 | 66.2 | 0 | 0.85 | 0.34 | heptyloxy magnesium 2.0 | |
| Example 10 | $I_2$, $MgCl_2$ | 1.5:1 | 0.01:1 | 13.0:1 | 10000 | 0 | n-butanol 200 | 56.6 | 0 | 0.76 | 0.32 | butoxy magnesium 2.0 | |
| Example 11 | $I_2$, $MgCl_2$ | 1.5:1 | 0.01:1 | 13.0:1 | 10000 | 300 | 200 | 76.8 | 0 | 0.65 | 0.39 | 1.5 | 1.2 |
| Example 12 | $I_2$, $MgCl_2$ | 1.5:1 | 0.01:1 | 13.0:1 | 10000 | 300 | 200 | 76.8 | 0 | 0.65 | 0.39 | 1.5 | 1.2 |
| Example 13 | $I_2$, $MgCl_2$ | 1.6:1 | 0.011:1 | 13.8:1 | 550 | 0 | 10 | 43.0 | 90 | 0.73 | 0.40 | 0 | 1.6 |
| Example 19 | $I_2$, $MgCl_2$ | 0.1:1 | 0.013:1 | 13.8:1 | 550 | 0 | 10 | 38.5 | 90 | 1.03 | 0.37 | 0 | 1.5 |
| Example 20 | $I_2$, $MgCl_2$ | 1:0.02 | 0.012:1 | 13.8:1 | 530 | 20 | 10 | 81.7 | 60 | 0.86 | 0.42 | 1.9 | 1.2 |
| Example 21 | $I_2$, $MgCl_2$ | 1:0.1 | 0.015:1 | 15.1:1 | 560 | 50 | 0 | 30 | 83.5 | 0.91 | 0.42 | 5.2 | 0 |
| Comparative Example 1 | $I_2$ | -- | 0.0068:1 | 16.4:1 | 13300 | 0 | 0 | 68.2 | 30 | 1.52 | 0.28 | 0 | 0 |
| Comparative Example 4 | $I_2$ | -- | 0.0018:1 | 16.4:1 | 13300 | 0 | 0 | 26.3 | | 1.05 | 0.22 | 0 | 0 |

Table 2 The data of catalyst and polypropylene

| item No. | Catalyst component | | | Propylene polymerization evaluation | | | | | |
|---|---|---|---|---|---|---|---|---|---|
| | Ti (wt%) | Catalyst component | | Ac (Kg/ gcat) | MWD | BD ($g/cm^3$) | MI (g/ 10min) | II (%) | Polymer fine powders <0.18μm (%) |
| | | Average particle size $D_{50}$ | Particle size distribution Span | | | | | | |
| Example 1 | 2.55 | 75.3 | 0.66 | 70.5 | 6.92 | 0.438 | 27.9 | 97.1 | 0.2 |
| Example 2 | 2.62 | 72.2 | 0.68 | 68.9 | 6.32 | 0.435 | 26.3 | 97.0 | 0.2 |
| Example 3 | 2.34 | 64.4 | 0.72 | 61.1 | 5.82 | 0.420 | 23.2 | 96.2 | 0.4 |
| Example 4 | 2.38 | 62.6 | 0.82 | 65.8 | 6.21 | 0.433 | 22.5 | 96.7 | 0.5 |
| Example 5 | 2.81 | 77.9 | 0.73 | 66.7 | 6.50 | 0.427 | 25.11 | 96.6 | 0.2 |
| Example 6 | 2.56 | 36.8 | 0.80 | 62.6 | 6.31 | 0.412 | 23.72 | 96.1 | 0.3 |
| Example 7 | 2.23 | 23.7 | 0.83 | 71.1 | 6.03 | 0.443 | 25.2 | 96.5 | 0.4 |
| Example 8 | 2.61 | 69.6 | 0.74 | 64.3 | 6.21 | 0.428 | 25.6 | 96.3 | 0.3 |
| Example 9 | 2.90 | 63.7 | 0.82 | 60.2 | 6.38 | 0.425 | 23.4 | 96.2 | 0.5 |
| Example 10 | 2.36 | 53.9 | 0.72 | 65.7 | 6.35 | 0.432 | 22.8 | 97.0 | 0.4 |
| Example 11 | 2.88 | 69.3 | 0.86 | 66.3 | 6.18 | 0.426 | 24.11 | 95.8 | 0.8 |
| Example 12 | 2.25 | 57.1 | 1.07 | 58.9 | 6.22 | 0.423 | 23.63 | 95.5 | 0.8 |
| Example 13 | 2.73 | 39.9 | 0.73 | 68.3 | 7.70 | 0.448 | 29.40 | 96.7 | 0.3 |
| Example 14 | 2.64 | 41.2 | 0.74 | 63.7 | 7.60 | 0.449 | 25.30 | 97.1 | 0.3 |
| Example 15 | 2.82 | 37.1 | 0.85 | 62.0 | 7.90 | 0.441 | 18.85 | 97.8 | 0.5 |
| Example 16 | 2.91 | 33.3 | 1.05 | 65.8 | 7.40 | 0.442 | 31.22 | 96.2 | 0.2 |
| Example 17 | 2.80 | 37.5 | 0.92 | 62.9 | 7.30 | 0.430 | 26.43 | 97.0 | 0.3 |
| Example 18 | 2.86 | 36.7 | 0.95 | 59.3 | 7.50 | 0.43 | 19.64 | 97.5 | 0.4 |
| Example 19 | 2.98 | 37.1 | 0.98 | 55.5 | 6.56 | 0.446 | 27.2 | 96.1 | 1.0 |
| Example 20 | 2.23 | 79.8 | 0.87 | 66.1 | 6.23 | 0.442 | 23.5 | 97.0 | 0.5 |
| Example 21 | 2.57 | 77.6 | 0.89 | 66.1 | 5.95 | 0.433 | 24.3 | 96.5 | 0.5 |
| Comparative Example 1 | 2.43 | 60.5 | 1.45 | 48.1 | 5.65 | 0.409 | 19.62 | 96.2 | 1.7 |
| Comparative Example 2 | 2.33 | 38.8 | 0.79 | 55.2 | 5.20 | 0.443 | 21.15 | 96.7 | 0.6 |
| Comparative Example 3 | 2.43 | 22.5 | 1.15 | 56.0 | 6.50 | 0.451 | 20.32 | 97.5 | 0.7 |

It can be seen from the data in Table 2, the catalyst obtained in the present invention has a high polymerization activity, a low content of polymer fine powders, a wide molecular weight distribution, and good isotactic and melt indexes. The polymers have good processing performances.

**Examples about propylene-ethylene block copolymers**

**Example 22**

(1) The preparation of a dialkoxy magnesium carrier

It is similar to Example 1.

(2)The preparation of a catalyst component

The steps are similar to those in Example 1.

(3) The olefin polymerization

The propylene and ethylene are used as said olefin, and are polymerized according to the following steps: In a 5L steel autoclave equipped with a stirrer, the air is fully replaced with nitrogen, which is then replaced with propylene, and then 5ml of hexane solution of triethyl aluminium (the concentration of triethyl aluminium is 0.5mmol/ml), 0.5ml of hexane solution of disopropyl dimethoxy silane (DIPDMS) (the concentration of DIPDMS is 0.10mmol/ml), 10ml of anhydrous hexane and 10mg of solid catalyst component are introduced at room temperature. The pressure is increased by hydrogen to 1.2MPa, and then 2.0L of liquid propylene is added. The temperature is increased to 70°Cunder stirring, and the polymerization is carried out at 70°C for 1h.

Then the stirring is stopped, the air is slowly emptied, and hot water is feed into the jacket at the same time to increase the temperature to 80°C. When the pressure in the reactor is decreased to 1.0MPa, the stir is started, and then the emptying and warming operation proceed slowly. When the temperature reaches 80°C, the pressure in the reactor is emptied to 0MPa (gauge pressure). Then 1.0 MPa of (gauge pressure) ethylene gas/propylene gas are feed in with a molar ratio of 3.2:6.8, and the propylene-ethylene copolymerization is carried out at 80 °C and 1.0 MPa for 1h. The pressure is released to the atmospheric pressure, and the temperature is decreased to room temperature. Then the reactor is opened, and the produced polymer particles are recovered. The results are shown in Table 3.

**Example 23**

Except that the molar ratio of ethylene to propylene in the second step is changed to 5.3:4.7, the others are similar to those in Example 11. The data are shown in Table 3.

**Comparative Example 4**

(1) The preparation of the dialkoxy magnesium carrier

After the air in a 16L pressure-resistant reactor equipped with a stirrer is fully replaced with nitrogen, 13300ml of ethanol and 6g of iodine are added into the reactor. The temperature is increased after stirring to reach the reflux temperature of the reaction system. Then a total of 640g of magnesium powders are successively added. The reaction is carried out until the reaction is completed, i.e. no hydrogen is discharged. Then the dialkoxy magnesium carrier is obtained after being washed, separated and dried. The data are shown in Table 1.

(2) The preparation of a catalyst component

Except that the above dialkoxy magnesium (1) is used, the other steps are similar to those in Example 1. The content of Ti is 2.42wt%, the average particle size is 25.1μm and SPAN is 1.01 in the obtained catalyst.

(3) The olefin polymerization

The steps are similar to those in Example 23. The data are shown in Table 3.

Table 3 Polymerization Data

| process | item | unit | Example 22 | Example 23 | Comparative Example 4 |
|---|---|---|---|---|---|
| First step | Polymerization time | min | 60 | 60 | 60 |
| | Polymerization temperature | °C | 70 | 70 | 70 |
| | Hydrogen pressure | MPa | 1.2 | 1.2 | 1.2 |
| | Total pressure | MPa | 3 | 3 | 3 |
| | Silicon compound | kind | DIPDMS | DIPDMS | DIPDMS |
| Second step | Polymerization time | min | 60 | 60 | 60 |
| | Polymerization temperature | °C | 80 | 80 | 80 |
| | $C_2$:$C_3$ | mol/mol | 3.2: 6.8 | 5.3: 4.7 | 5.3: 4.7 |
| | Total pressure | MPa | 1 | 1 | 1 |
| | Polymerization activity | KgPP/gC at | 6.65 | 6.92 | 5.28 |
| | MI | g/10min | 5.52 | 6.26 | 7.02 |
| | XS | wt% | 13.50 | 15.77 | 17.62 |
| | $C_2$ | mol % | 15.32 | 19.51 | 16.30 |

It can be seen from Table 3 that, the copolymers with a high ethylene content can be obtained with a low content of ethylene raw gas by using the catalyst provided by the present invention. The copolymerization performances of said catalyst are good, and the application prospect is good.

## Claims

1. A method of preparing dialkoxymagnesium carrier comprising the following steps:

   magnesium and mixed alcohols are used as raw materials and a reflux reaction is performed in the presence of mixed halogenated agents under an inert atmosphere,
   said mixed halogenated agent being iodine and magnesium chloride, and the weight ratio of said iodine to magnesium being 0.05:1 to 1:0.01.

2. The method according to claim 1, wherein the weight ratio of iodine to magnesium is 0.1:1 to 1:0.02.

3. The method according to claim 1 or 2, wherein said alcohols comprise ethanol and at least one selected from $C_6$-$C_{11}$ alcohols, preferably said alcohols comprise ethanol and at least one selected from $C_6$-$C_8$ alcohols, more preferably said alcohols are ethanol and 2-ethyl hexanol, and in said carrier, the content of ethoxy magnesium is equal to or higher than 80wt%, and the content of 2-ethyl hexyloxy magnesium is 0.01 to 20wt%.

4. The method according to any one of the claims 1 to 2, wherein said alcohols comprise ethanol and at least one selected from $C_1$-$C_5$ lower alcohols, and said lower alcohols do not contain ethanol, preferably said alcohols comprise ethanol and isopropanol, and in said carrier, the content of ethoxy magnesium is equal to or higher than 80wt%, and the content of isopropoxy magnesium is 0.01 to 20wt%.

5. The method according to claim 1 or 2, wherein said alcohols comprise ethanol, at least one selected from $C_6$-$C_{11}$ alcohols and at least one selected from $C_1$-$C_5$ lower alcohols, said lower alcohol containing no ethanol, preferably said alcohols comprise ethanol, 2-ethyl hexanol and isopropanol, and in said carrier, the content of ethoxy magnesium is equal to or higher than 80wt%, the content of 2-ethyl hexyloxy magnesium is 0.01 to 19.9wt% and the content of isopropoxy magnesium is 0.01 to 19.9wt%.

6. The method according to any one of claims 1 to 5, wherein the weight ratio of said alcohols and magnesium is 4:1 to 50:1, and the molar ratio of said magnesium and the halogen atoms in said mixed halogenated agent is 1:0.0002 to 1:0.2, preferably the weight ratio of said alcohols and magnesium is 6:1 to 25:1, and the molar ratio of said

magnesium and the halogen atoms in said mixed halogenated agent is 1:0.001 to 1:0.08.

7. The method according to any one of the claims 1 to 6, wherein the average particle size of dialkoxy magnesium is 5 to 150μm, and the particle size distribution index SPAN thereof is less than 1.1, preferably the average particle size of dialkoxy magnesium is 8 to 100μm, and the particle size distribution index SPAN thereof is less than 1.05.

8. Use of the method according to any one of the claims 1 to 7, for the production of a solid catalyst component for olefin polymerization.

**Patentansprüche**

1. Verfahren zur Herstellung eines Dialkoxymagnesiumträgers, enthaltend die folgenden Schritte:

   Magnesium und gemischte Alkohole werden als Ausgangsmaterialien verwendet und eine Rückflussreaktion in der Gegenwart der gemischten halogenierten Mittel unter einer Inertatmosphäre durchgeführt,
   das gemischte halogenierte Mittel ist Jod und Magnesiumchlorid, und das Gewichtsverhältnis von Jod zu Magnesium ist 0,05:1 bis 1:0,01.

2. Verfahren gemäß Anspruch 1, worin das Gewichtsverhältnis von Jod zu Magnesium 0,1:1 bis 1:0,02 ist.

3. Verfahren gemäß Anspruch 1 oder 2, worin die Alkohole Ethanol und zumindest einen enthalten, ausgewählt aus $C_{6-11}$-Alkoholen, worin bevorzugt die Alkohole Ethanol und zumindest einen, ausgewählt aus der Gruppe, bestehend aus $C_{6-8}$-Alkoholen enthalten, mehr bevorzugt die Alkohole Ethanol und 2-Ethylhexanol sind und in dem Träger der Gehalt von Ethoxymagnesium gleich oder mehr als 80 Gew.-% ist und der Gehalt von 2-Ethylhexyloxymagnesium 0,01 bis 20 Gew.-% ist.

4. Verfahren gemäß einem der Ansprüche 1 bis 2, worin die Alkohole Ethanol und zumindest einen, ausgewählt aus $C_{1-5}$-Niedrigalkoholen enthalten und die Niedrigalkohole keinen Ethanol enthalten, bevorzugt die Alkohole Ethanol und Isopropanol enthalten und in dem Träger der Gehalt von Ethoxymagnesium gleich oder höher als 80 Gew.-% und der Gehalt von Isopropoxymagnesium 0,01 bis 20 Gew.-% ist.

5. Verfahren gemäß Anspruch 1 oder 2, worin die Alkohole Ethanol, zumindest einen, ausgewählt aus $C_{6-11}$-Alkoholen und zumindest einen, ausgewählt aus $C_{1-5}$-Niedrigalkoholen enthalten, wobei der Niedrigalkohol keinen Ethanol enthält, bevorzugt die Alkohole Ethanol, 2-Ethylhexanol und Isopropanol enthalten und in dem Träger der Gehalt von Ethoxymagnesium gleich oder höher als 80 Gew.-% ist, der Gehalt von 2-Ethylhexyloxymagnesium 0,01 bis 19,9 Gew.-% ist und der Gehalt von Isopropoxymagnesium 0,01 bis 19,9 Gew.-% ist.

6. Verfahren gemäß einem der Ansprüche 1 bis 5, worin das Gewichtsverhältnis der Alkohole und Magnesium 4:1 bis 50:1 ist und das molare Verhältnis von Magnesium und den Halogenatomen im gemischten halogenierten Mittel 1:0,0002 bis 1:0,2 ist, bevorzugt das Gewichtsverhältnis der Alkohole und Magnesium 6:1 bis 25:1 ist und das molare Verhältnis von Magnesium und den Halogenatomen in dem gemischten halogenierten Mittel 1:0,001 bis 1:0,08 ist.

7. Verfahren gemäß einem der Ansprüche 1 bis 6, worin die durchschnittliche Teilchengröße von Dialkoxymagnesium 5 bis 150 μm ist und der Teilchengrößenverteilungsindex SPAN davon weniger als 1,1 ist, bevorzugt die durchschnittliche Teilchengröße von Dialkoxymagnesium 8 bis 100 μm und der Teilchengrößenverteilungsindex SPAN davon weniger als 1,05 ist.

8. Verwendung des Verfahrens gemäß einem der Ansprüche 1 bis 7 zur Herstellung einer festen Katalysatorkomponente für die Olefinpolymerisation.

**Revendications**

1. Procédé de préparation d’un support dialkoxymagnésium comprenant les étapes suivantes :

   du magnésium et des alcools mélangés sont utilisés en tant que matériaux bruts et une réaction de reflux est

réalisée en présence d'agents halogénés mélangés sous une atmosphère inerte,
ledit agent halogéné mélange étant de l'iode et du chlorure de magnésium, et le rapport en poids de ladite iode au magnésium étant de 0,05:1 à 1:0,01.

**2.** Le procédé selon la revendication 1, dans lequel le rapport en poids de l'iode au magnésium est de 0,1:1 à 1:0,02.

**3.** Le procédé selon la revendication 1 ou 2, dans lequel lesdits alcools comprennent de l'éthanol et au moins l'un choisi parmi des alcools en $C_6$-$C_8$, plus préférablement lesdits alcools sont de l'éthanol et du 2-éthyl hexanol, et dans ledit support, la teneur d'éthoxy magnésium est égale à ou supérieure à 80% en poids, et la teneur de 2-éthyl hexyloxy magnésium est 0,01 à 20% en poids.

**4.** Le procédé selon l'une quelconque des revendications 1 à 2, dans lequel lesdits alcools comprennent de l'éthanol et au moins l'un choisi parmi des alcools inférieurs en $C_1$-$C_5$, et lesdits alcools inférieurs ne contiennent pas d'éthanol, préférablement lesdits alcools comprennent de l'éthanol et l'isopropanol, et dans ledit support, la teneur d'éthoxy magnésium est égale à ou supérieure à 80% en poids, et la teneur d'isopropoxy magnésium est de 0,01 à 20% en poids.

**5.** Le procédé selon la revendication 1 ou 2, dans lequel lesdits alcools comprennent de l'éthanol, au moins l'un choisi parmi des alcools en $C_6$-$C_{11}$ et au moins l'un choisi parmi des alcools inférieurs en $C_1$-$C_5$, ledit alcool inférieur ne contenant pas d'éthanol, préférablement lesdits alcools comprennent de l'éthanol, du 2-éthyl hexanol et de l'isopropanol, et dans ledit support, la teneur d'éthoxy magnésium est égale à ou supérieure à 80% en poids, la teneur de 2-éthyl hexyloxy magnésium est de 0,01 à 19,9% en poids et la teneur d'isopropoxy magnésium est de 0,01 à 19,9% en poids.

**6.** Le procédé selon l'une quelconque des revendications 1 à 5, dans lequel le rapport en poids desdits alcools et du magnésium est de 4:1 à 50:1, et le rapport molaire dudit magnésium et des atomes d'halogène dans ledit agent halogéné mélangé est de 1:0,0002 à 1:0,2, préférablement le rapport en poids desdits alcools et du magnésium est de 6:1 à 25:1, et le rapport en poids dudit magnésium et des atomes d'halogène dans ledit agent halogéné mélangé est de 1:0,001 à 1:0,08.

**7.** Le procédé selon l'une quelconque des revendications 1 à 6, dans lequel la taille de particule moyenne de dialkoxy magnésium est de 5 à 150μm, et l'indice de distribution de taille de particule SPAN de celui-ci est inférieur à 1,1, préférablement la taille de particule moyenne de dialkoxy magnésium est de 8 à 100μm, et l'indice de distribution de taille de particule SPAN de celui-ci est inférieur à 1,05.

**8.** Utilisation du procédé selon l'une quelconque des revendications 1 à 7, pour la production d'un composant de catalyseur solide pour la polymérisation d'oléfines.

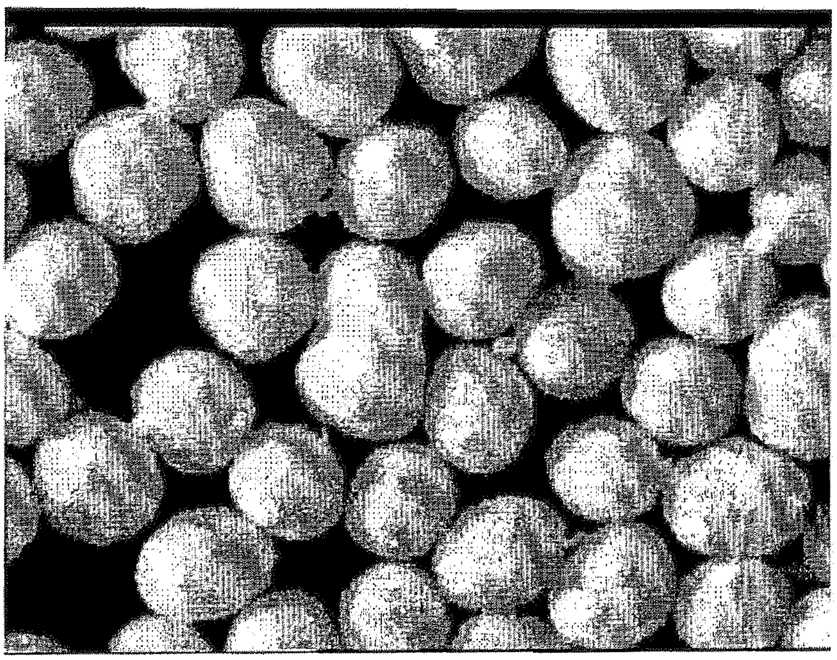

Figure 1

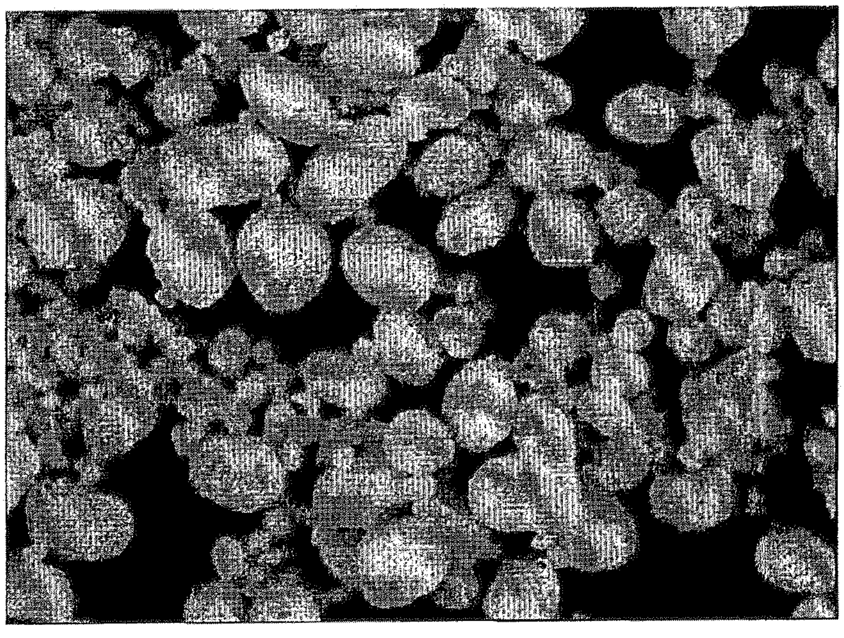

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 85100997 A
- CN 1453298 A
- JP 3001991 A
- JP 74341 A
- JP 6001994 A
- JP 87773 A
- JP 63004815 A
- CN 1875038 A
- EP 0459009 A
- EP 0811639 A
- CN 101054424 A
- CN 1436766
- CN 1436796 A
- GB 36822000 T